# EUROPEAN PATENT APPLICATION

(11) **EP 3 628 312 A1**
(43) Date of publication of application: **01.04.2020**
(21) Application number: 18197538.4
(22) Date of filing: 28.09.2018
(51) Int. Cl.: A61K 31/045, A61K 33/06, A61P 3/14

(54) **CALCIUM PREPARATION**

(71) Applicant: PerformaNat GmbH, 14163 Berlin (DE)
(72) Inventor: Rosendahl, Julia, 10719 Berlin (DE); Schrapers, Katharina, 14195 Berlin (DE); Braun, Hannah-Sophie, 12163 Berlin (DE)

(57) **Abstract**

The present invention pertains to a composition comprising a calcium source, a TRP agonist and water.

## Description

The present invention pertains to composition comprising a calcium source and water.

Such compositions are known from US 5,395,622 and WO 2006/072252, which disclose preparations comprising a combination of calcium chloride and calcium sulphate. The commercially available solid bullets with tradename BoviKalc® contains 57.9% CaCl₂.6H2O and 23.2% CaSO₄.1/2H₂O and 14.4% water with a coating of glyceryl PEG ricinoleate (4.5%). Such solid bullets are used to treat animals with milk fever, and are administered to the animal with a bullet gun, that allows the bullet to be swallowed into the rumen without risk for inhalation into the respiratory tract or lungs. The uptake of calcium in the conventional composition or preparations is still limited. Administration of conventional calcium preparations may lead to mucosal damage and/or increased systemic toxin uptake through the rumen.

It is an object of the invention to provide for an improved composition.

The invention pertains to Sa composition comprising a calcium source, a TRP agonist and water, wherein the amount of the calcium source is at least 5 wt%, based on the total weight of the composition. The TRP agonist of the invention enables the calcium to be taken up in the rumen more easily by increasing the calcium flux through the mucosal wall, therewith increasing the calcium levels in the blood plasma of the animal, preferably of the cow, faster than conventional compositions comprising a calcium source. The presence of the TRP agonist also allows compositions with a lower amount of calcium compared to conventional compositions. Additionally and/or alternatively, the number of administrations to the animal, preferably the cow, can be reduced. This lower number of administrations may further reduce the risk of erosive or irritative effect on the mucosa of the stomach of the animal. Moreover, the use of the TRP agonist was shown to reduce the paracellular flux of the mucosal epithelium which causes the mucosal epithelial cells to be less prone to damage, which in turn leads to a lower occurrence of acidosis or other disease associated with administration of calcium preparations and/or a lower systemic toxin uptake through the rumen. The simultaneous effect of an increased calcium flux and a decreased paracellular flux was found to be more pronounced with calcium amounts exceeding 10 wt%, preferably exceeding 15 wt%, based on the total weight of the composition.

The composition of the invention comprises a calcium source. The calcium source can be any calcium source known in the art. Examples of such calcium sources include calcium chloride, calcium sulphate, calcium propionate, calcium formate, calcium lactate, calcium carbonate, monocalcium phosphate, calcium acetate and combinations of two or more calcium sources. Preferably, the calcium source is selected from the group consisting of calcium chloride, calcium propionate and calcium formate. Most preferred is calcium formate.

The composition of the invention generally comprises the calcium source in an amount of at least 5 percent by weight (wt%). Preferably, the amount of calcium source is at least 8 wt%, more preferably at least 10 wt%, even more preferably at least 15 wt%, and most preferably at least 20 wt%, and preferably at most 85 wt%, more preferably at most 80 wt%, even more preferably at most 75 wt%, and most preferably at most 70 wt%, based on the total weight of the composition. The amount of the calcium source is based on the weight of the calcium salt including crystal water, and in case of a combination of two or more calcium sources is based on the total weight of all the calcium salts including crystal water.

The composition of the invention generally comprises calcium in an amount of at least 5 percent by weight (wt%). Preferably, the amount of calcium source is at least 8 wt%, more preferably at least 10 wt%, even more preferably at least 15 wt%, and most preferably at least 20 wt%, and preferably at most 40 wt%, more preferably at most 35 wt%, even more preferably at most 30 wt%, and most preferably at most 25 wt%, based on the total weight of the composition. The amount of calcium is determined by the actual weight of the Ca²⁺ ions present in the calcium salt, and in case of a combination of two or more calcium sources is based on the total weight of the Ca²⁺ ions present in the calcium salts.

The composition of the invention comprises a TRP agonist. The term "TRP agonist" refers to compounds that can increase the activity of the transient receptor potential (TRP) channels. The TRP agonist can influence one or more specific TRP channels. For instance, the TRP agonist may activate or increase the activity of two or more TRP channels. The TRP agonist may activate one or more TRP channels and may inhibit another TRP channel. Examples of such TRP channels include TRPV (vanilloid receptor) such as TRPV1, TRPV2, TRPV3, TRPV4, TRPV5 and TRPV6; TRPM (melastatin) such as TRPM3, TRPM5, TRPM6, TRPM7 and TRPM8; TRPC (classic) such as TRPC1, TRPC3 and TRPC6; TRPA (ankyrin) such as TRPA1; TRPP (polycystin) such as TRPP1, TRPP2 and TRPP3; and TRPML (mucolipin) such as TRPML1, TRPML2 and TRPML3. Especially interesting for the composition of the invention are the TRP channels present in ruminants. In the context of the present application, the TRP agonist effectively increases the uptake of Ca²⁺ ions in the rumen leading to an effective increase of Ca²⁺ ions in the blood plasma of the animal, preferably the cow, when compared to the Ca²⁺ levels in plasma of compositions without the TRP agonist. Examples of TRP modulators include capsaicin, capsiate, capsaicinoids, menthol, eucalyptol, resiniferatoxin, resinifernaoids, piperine, piperidine, camphor, bisandrographolide, 2-aminoethyl-diphenylborinate, icilin, olvanil, verapamil, quinidine, GsMTx4, St. John's wort, hyperforin, ginger, vanillylacetone, evodiamine, gingerol, shogaol, other shogaols, cannabinol, cannabidiol, cannabis, tetrahydrocannabinol, other cannabinoids, polygodal, drimanial, cinnamodial, cinnamosmolide, cinnamolide, afromodial, ancistrodial, merulidial, grifolin, neogrifolin, albaconol, o-prenylphenol, (N-(4-tertiarybutylphenyl)-4-(3-chloropyridin-2-yl)-tetrahydro-pyrazin-1(2H)-carboxamide, isovelleral, vanillin, vanllotoxin 1, 2 or 3, arvanil, cnidarian envenomations, yohimbine, AG489 toxin, AG505 toxin, paradol, ginsenoside, mustard oil, allyl isothiocyanate (wasabi), carvacrol, carveol, thymol, borneol, menthone, geraniol, oleocanthal, linalool, isomenthon, menthyl lactate, anethol, p-menthane-3,8-diol, 1-carvone, d-carvone, isopulegol, hydroxyl-citronellal, allicin, cinnamaldehyde (cinnamic aldehyde), methyl salicilate, benzyl isothiocyanate, phenylethyl isothiocyanate, isopropyl isothiocyanate, methyl isothiocyanate, prostaglandin, prostaglandin synthesis inhibitors, acetylsalicylic acid, paracetamol, ibuprofen, warburganal, and combinations of two or more TRP agonist. In one embodiment, the TRP agonist is selected from the group consisting of menthol, thymol, cinnamaldehyde, eugenol, anethol, carvacrol, capsaicin, cannabidiol, isomenthon, vanillin, linalool, cannabinol, cannabis, citral and oleocanthal. In another embodiment, the TRP agonist is selected from the group consisting of menthol, thymol, cinnamaldehyde, eugenol, anethol, isomenthon, carvacrol, vanillin and linalool. In a preferred embodiment, the TRP agonist is selected from the group consisting of menthol, thymol, cinnamaldehyde, carvacrol, vanillin and linalool. More preferably, the TRP agonist is selected from the group consisting of menthol, thymol, cinnamaldehyde and carvacrol. In a further preferred embodiment, the TRP agonist is selected from the group consisting of menthol, thymol and cinnamaldehyde. Even more preferably, the TRP agonist is selected from menthol and thymol. Most preferably, the TRP agonist is menthol.

In one embodiment, the composition of the invention may comprise the TRP agonist in an amount of at least 0.01 percent by weight (wt%), preferably at least 0.05 wt%, more preferably at least 0.1 wt%, even more preferably at least 0.15 wt%, and most preferably at least 0.2 wt%, and preferably at most 10 wt%, more preferably at most 8 wt%, even more preferably at most 5 wt%, even more preferably at most 2 wt%, and most preferably at most 1 wt%, based on the total weight of the composition.

The composition of the invention further comprises water. In one embodiment, the composition of the invention may comprise water in an amount of at least 5 percent by weight (wt%), preferably at least 10 wt%, more preferably at least 15 wt%, even more preferably at least 20 wt%, and most preferably at least 25 wt%, and preferably at most 90 wt%, more preferably at most 80 wt%, even more preferably at most 70 wt%, even more preferably at most 60 wt%, and most preferably at most 50 wt%, based on the total weight of the composition.

The composition of the invention may further comprise an excipient. The excipient can be any excipient known in the art and used in composition with relatively high amounts of calcium. The excipient of the invention may be a binder, a metal source (other than calcium source) and a vitamin, for example. Combinations of two or more excipients are contemplated.

The binder can be any binder suitably used in compositions of the invention. Such binders may improve the integrity of pre-shaped solid preparations like a bullet. In such case the binder may obviate the use of a (bullet) coating or a capsule. Preferably, the binders of the invention are approved for use in animal feed. Example of suitable binders include starches like potato starch, modified cellulose such as carboxymethyl cellulose (CMC) and hydroxypropyl methoxy cellulose (HPMC) and polyethelene glycols (PEG) such as PEG 2000 and PEG 6000.

The metal source can be any metal source known in the art, which is different from the calcium source. Examples of suitable metal sources include magnesium sources like magnesium oxide and magnesium citrate and phosphorous sources like sodium phosphate, dicalcium phosphate and bone meal.

The vitamins can be any vitamin known in the art that can be used in animal feed. Examples of such vitamins include vitamin A, vitamin C, vitamin D such vitamin D3 and vitamin E.

The excipient is optional and does not need to be present in the composition. In one embodiment, the composition of the invention may comprise the excipient in an amount of at least 1 percent by weight (wt%), preferably at least 2 wt%, more preferably at least 5 wt%, even more preferably at least 10 wt%, and most preferably at least 15 wt%, and preferably at most 60 wt%, more preferably at most 50 wt%, even more preferably at most 40 wt%, even more preferably at most 30 wt%, and most preferably at most 25 wt%, based on the total weight of the composition.

The composition of the invention can be in any form. The composition can be liquid or solid. Preferably, the composition is a pre-shaped solid preparation such as a bullet. Consequently, the invention pertains to a pre-formed or pre-shaped solid preparation comprising a calcium source, a TRP agonist, water and optionally an excipient, wherein the amount of the calcium source is at least 5 wt%, based on the total weight of the composition.

In one embodiment, the pre-formed solid preparation is coated with a water-soluble coating composition. The water-soluble coating composition can be any coating composition known in the art. Examples of suitable coating composition include glyceryl PEG ricinoleate, polyvinyl pyrrolidone (PVP) and polylactic acid (PLA). Preferably, the coating composition comprises glyceryl PEG ricinoleate.

In one embodiment, the pre-formed solid preparation of the invention may comprise the coating composition in an amount of at least 0.1 percent by weight (wt%), preferably at least 0.2 wt%, more preferably at least 0.5 wt%, even more preferably at least 1 wt%, and most preferably at least 1.5 wt%, and preferably at most 20 wt%, more preferably at most 15 wt%, even more preferably at most 10 wt%, even more preferably at most 8 wt%, and most preferably at most 5 wt%, based on the total weight of the composition.

The invention further pertains to the use of the composition according to the invention or the pre-formed composition according to the invention to treat hypocalcaemia in ruminants, preferably cows.

The invention is illustrated with the following examples.

### EXAMPLES

### Examples 1 and 2 and Comparative Example A

### Measurement of Ca-Flux across ruminal epithelium in the Ussing-chamber

Ruminal epithelium of sheep was mounted in a conventional Ussing chamber. Chambers contained 16 ml of serosal or mucosal buffer, which was circulated and gassed by a gas lift system (5% CO2/95% O₂).

### Groups

In this experiment 24 chambers were divided into three groups. Group 1 (mucosal buffer containing 1.8mM CaCl₂), group 2 (mucosal buffer containing 3.6mM CaCl) and group 3 (containing 7.2mM CaCl₂). The same buffer was present on the serosal side of all chambers (Table 1). Each group (1,2,3) was again divided into two parts. One was defined as control group one as EO group. It is noted that the amounts correspond to a normal Ca²⁺ concentration in the rumen (1.8 mM) (referred to as Comparative Example A) and to treated rumen corresponding to the administration of about 13 g Ca (3.6 mM) and 26 g Ca (7.2 mM) (referred to as Examples 1 and 2, respectively).

### Procedure

After an equilibrating time of around 30 minutes ⁴⁵Ca²⁺ (40kBq per 16 ml chamber) was added to either to the mucosal or serosal "hot" side.

Depending on the group 16*µ*l ethanol (Control) or 16*µ*l essential oil (EO) were added to the mucosal side of the chamber. The essential oil used is menthol, and the actual menthol concentration obtained is 50 µM.

After 70 minutes the first sample from the "cold" side was taken, followed by five samples of each 600*µ*l which were taken in an interval of 30 minutes. The sample volume was replaced by an aliquot of the respective buffer. Radioactivity was measured using a β-counter (LKB Wallace-Perkin-Elmer, Überlingen, Germany).

Epithelia were paired after sorting for transepithelial conductance (Gt) and the net calcium flux was calculated from the difference between mucosal to serosal flux and serosal to mucosal flux for paired epithelia.

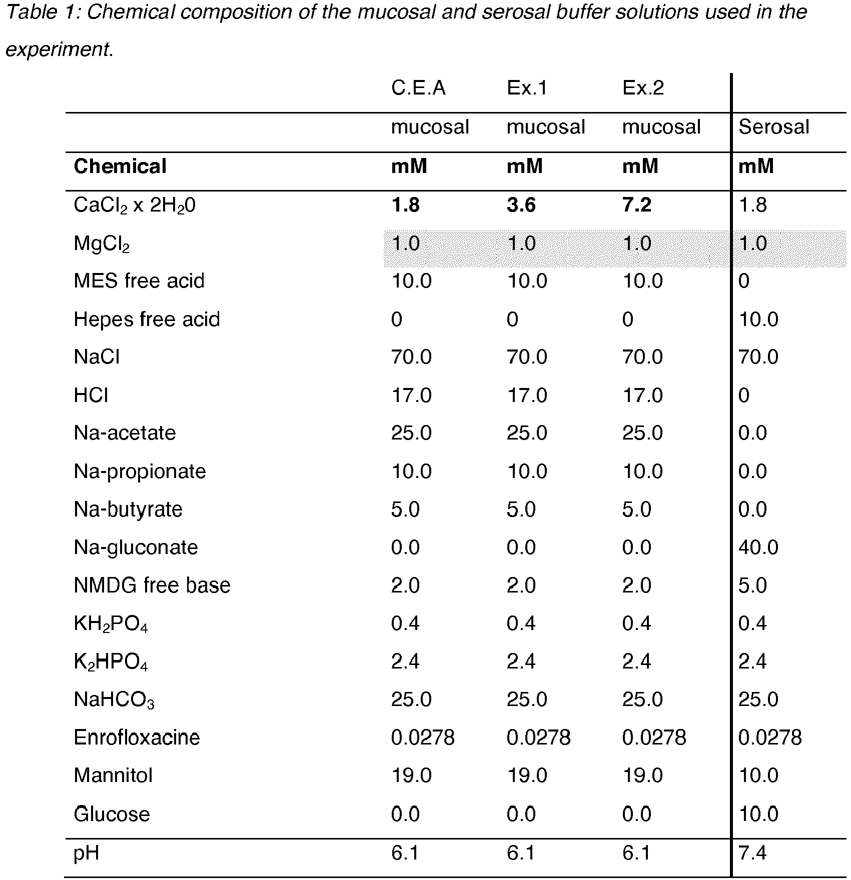

The net transport of calcium across the isolated ruminal epithelium was calculated by subtracting the calcium secretion (serosal to mucosal transport) from the calcium absorption (mucosal to serosal transport) reflecting the real absorption of calcium from the rumen to the blood side.

For statistical analysis linear mixed effect models for repeated measures were calculated using mucosal calcium concentration and the addition of essential oils as fixed effects and animal as random effect (SAS System, method MIXED PROC). The effects of calcium concentration and the addition of essential oils was calculated as well as their interaction. Furthermore, the effect of sampling time was included to the model. Effects were evaluated for the repeated measures of the consecutive flux periods.

### Results

The addition of EO increases Calcium net flux as it was already shown in former studies. Surprisingly it is also possible to increase calcium net flux even under high calcium conditions.

**Table 2: Shown are the mean and SEM of measured Ca flux across ruminal epithelium depending on mucosal calcium concentration and the presence or absence of EO.**

| Example | N/n=13/312 | Control | | EO | | | |
|---|---|---|---|---|---|---|---|
| | Ca | Mean (nmol/cm²/h) | SEM | Mean (nmol/cm²/h) | SEM | Difference (nmol/cm²/h) | P-value |
| A | 1.8 | 22.18 | 2.22 | 28.24 | 2.28 | +6.06 | <0.05 |
| 1 | 3.6 | 43.25 | 2.17 | 45.77 | 2.26 | +2.52 | n.s. |
| 2 | 7.2 | 67.00 | 2.13 | 75.22 | 2.39 | +8.22 | <0.05 |

The net calcium absorption significantly increased with increasing mucosal calcium concentrations. Furthermore, the addition of EO increased calcium absorption by 6.06, 2.52 and 8.22 nmol/cm²/h for the increasing mucosal calcium concentration. There was no significant interaction between the factors calcium concentration and EO addition.

The calcium flux is increased at all three Ca concentrations when menthol is present compared to the control. The relative flux (i.e. the percentage measured against the control flux) is above 100% for all menthol.-containing preparations, with a minimum at a Ca concentration of 3.6 mM

### Examples 3 and 4 and Comparative Example B

### Effect of EO on cellular barrier integrity of ruminal epithelium in Ussing-chamber depending on mucosal calcium concentration

Ruminal epithelium of sheep was mounted in a conventional Ussing chamber. Chambers contained 16 ml of serosal or mucosal buffer, which was circulated and gassed by a gas lift system (5% CO₂/95% O₂).

### Groups

In this experiment 24 chambers were divided into three groups. Group 1 (mucosal buffer containing 1.8mM CaCl₂), group 2 (mucosal buffer containing 3.6mM CaCl₂) and group 3 (containing 7.2mM Cacl). The same buffer was present on the serosal side of all chambers (Table 1). Each group (1,2,3) was again divided into two parts. One was defined as control group one as EO group. It is noted that the amounts correspond to a normal Ca²⁺ concentration in the rumen (1.8 mM) (referred to as Comparative Example B) and to treated rumen corresponding to the administration of about 13 g Ca (3.6 mM) and 26 g Ca (7.2 mM) (referred to as Examples 3 and 4, respectively).

### Procedure

Fluorescein is a well-known marker for paracellular permeability. To measure net fluorescein flux rates (***J*_{net-fluor=}J_{ms-fluor-}J_{sm-fluor}**), 16*µ*l sodium fluorescein was added to a final concentration of 0.1 m*M* to the buffer solution on the "hot" side of each tissue after the equilibration period of 30 min. Epithelia were paired after sorting for transepithelial conductance (Gt) and the fluorescein flux was measured from the mucosal to serosal side. To determine the specific fluorescence intensity, 50*-µ*L samples were taken from the "hot" side of each tissue immediately after adding. Depending on the group CON (16*µ*l ethanol) or EO (16*µ*l EO) were added to the mucosal side of the chamber.

After 70 minutes the first sample from the "cold" side was taken, followed by five samples of each 300*µ*l which were taken in an interval of 30 minutes. The sample volume was replaced by an aliquot of the respective buffer. Fluorescence was measured in a plate reader at 490 nm excitation and 525 nm emission (EnSpire Multimode Plate Reader, Perkin Elmer, Waltham, MA).

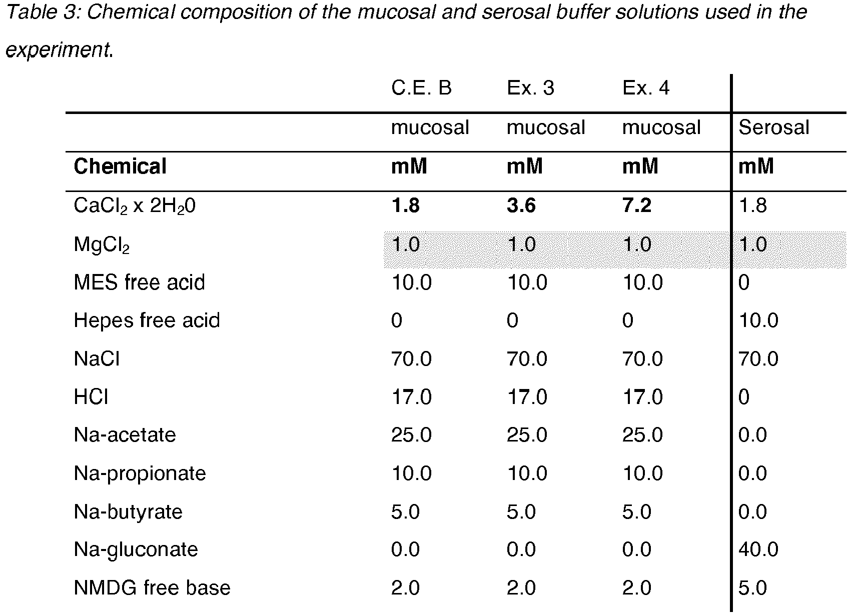

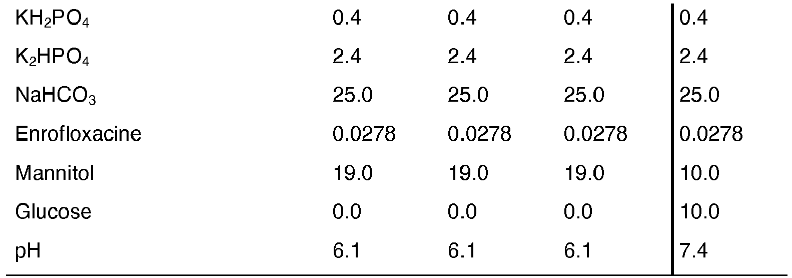

Statistics:
All results are given as mean ± SEM. P values of < 0.05 were considered to be significant.
N refers to the number of animals, n refers to the number of individual measurements of tissues. Multiple comparisons were performed using one-way (repeated measures) ANOVA using Sigma Plot program for Windows.

### Results

The paracellular flux across ruminal epithelium under three different calcium concentrations were measured. The results are shown in Table 4 below.

**Table 4: Shown are the mean and SEM of measured paracellular fluxrates across ruminal epithelium depending on mucosal calcium concentration and the presence or absence of EO.**

| Example | N/n= 13/312 | Ca-conc. mM | Fluorescein flux nmol/h/cm² | | Rel. flux % | P-value |
|---|---|---|---|---|---|---|
| | | | Mean | SEM | | |
| | Con | 1.8 | 0.072 | 0.027 | 100 | |
| B | EO | 1.8 | 0.068 | 0.025 | 93.5 | n.s. |
| | Con | 3.6 | 0.082 | 0.030 | 100 | |
| 3 | EO | 3.6 | 0.051 | 0.025 | 91.8 | n.s. |
| | Con | 7.2 | 0.005 | 0.031 | 100 | |
| 4 | EO | 7.2 | -0.045 | 0.041 | 75.9 | <0.05 |

The paracellular flux is considerably reduced with the addition of menthol at high Ca concentration (Example 4) which is according to the invention. Under such conditions (i.e. high Ca concentrations), paracellular barrier integrity of the rumen mucosa is significantly increased, whereby the rumen mucosa is likely less affected by the calcium preparation of the invention. The paracellular flux observed for Example 3 and Comparative Example B are not significantly different from the control.

## Claims

1. Composition comprising a calcium source, a TRP agonist, water and optionally an excipient, wherein the amount of the calcium source is at least 5 wt%, based on the total weight of the composition.

2. Composition according to claim 1 comprising 5 to 85 wt% calcium source and 0.01 to 10 wt% TRP agonist, 5 to 90 wt% water and 0 to 50 wt% excipient, based on the total weight of the composition.

3. Composition according to any one of claims 1 and 2, wherein the excipient is a binder.

4. Pre-formed solid preparation comprising the composition according to any one of the preceding claims.

5. Pre-formed solid preparation according to claim 4, wherein the pre-formed solid preparation is coated with a water-soluble coating composition.

6. Use of the composition according to any one of claims 1 to 3 or the pre-formed composition according to any one of claims 4 and 5 to treat hypocalcaemia in ruminants, preferably cows.
